# EUROPEAN PATENT APPLICATION

(11) **EP 4 767 963 A1**
(43) Date of publication of application: **01.07.2026**
(21) Application number: 26150053.2
(22) Date of filing: 02.01.2026
(51) Int. Cl.: A61B 18/12, A61B 17/32, A61B 18/14

(54) **TECHNOLOGIES FOR POWER DELIVERY FOR ENERGY-BASED SURGICAL INSTRUMENTS**

(30) Priority: 31.12.2024 US 202463740950 P; 30.09.2025 US 202519345323
(71) Applicant: Cilag GmbH International, 6300 Zug (CH)
(72) Inventor: SHELTON, IV, Frederick E., Cincinnati, 45242 (US); HARRIS, Jason L., Cincinnati, 45242 (US)
(74) Representative: Carpmaels & Ransford LLP

(57) **Abstract**

A system includes an energy-based surgical instrument with an end effector having two electrodes. One of the electrodes has multiple electrode portions. A generator coupled to the surgical instrument supplies radio-frequency (RF) energy to an electrode portion for a tissue coagulation process and further supplies RF energy at a higher voltage or current to another electrode portion for a tissue transection process. A control element may monitor tissue impedance or tissue temperature. Other embodiments are described and claimed.

## Description

### CROSS-REFERENCE TO RELATED APPLICATION

This application claims the benefit of and priority to U.S. Patent Application No. 63/740,950, entitled "TECHNOLOGIES FOR OPTIMIZED POWER DELIVERY FOR ENERGY-BASED SURGICAL INSTRUMENTS," which was filed on December 31, 2024, and which is incorporated herein by reference in its entirety.

### TECHNICAL FIELD

The present disclosure relates generally to energy-based surgical instruments and, more particularly, to harmonic and/or electrosurgical surgical instruments.

### BACKGROUND

Energy-based surgical instruments are finding increasingly widespread applications in surgical procedures by virtue of their unique performance characteristics. Depending upon specific device configurations and operational parameters, energy-based surgical instruments can provide both transection of tissue and hemostasis of the tissue by coagulation, which may reduce or otherwise minimize patient trauma. Depending on the particular application, energy-based surgical instruments may utilize different surgical technologies including, for example, ultrasonic and/or electro-surgical (e.g., radio frequency (RF)) technologies.

A typical ultrasonic surgical instrument may include a handpiece containing an ultrasonic transducer and an elongated shaft assembly having a distally mounted end effector to effect the cutting and sealing of tissue. For example, the end effector may include a jaw assembly having an ultrasonic blade and a clamp arm, which may include a non-stick tissue pad or similar bed to receive the ultrasonic blade. In some cases, the elongated shaft assembly may be permanently affixed to the handpiece. In other cases, the elongated shaft assembly may be detachable from the handpiece, as in the case of a disposable shaft assembly or a shaft assembly that is interchangeable between different handpieces. In use, the end effector transmits ultrasonic energy to tissue brought into contact with the ultrasonic blade of the end effector to realize the cutting and sealing action. Such ultrasonic surgical devices may be configured for open surgical use, laparoscopic, and/or endoscopic surgical procedures including robotic-assisted procedures.

Ultrasonic energy cuts and coagulates tissue using temperatures lower than those used in electro-surgical procedures. Vibrating at high frequencies (e.g., 55,500 times per second), the ultrasonic blade denatures protein in the tissue to form a sticky coagulum. Pressure exerted on tissue by the ultrasonic blade surface collapses blood vessels and allows the coagulum to form a hemostatic seal. A surgeon can control the cutting speed and coagulation by the force applied to the tissue by the end effector, the time over which the force is applied, and the selected excursion level of the end effector.

In electro-surgical instruments, one or more electrodes are incorporated into the end effector and configured to apply therapeutic electrical current to the patient's tissue to create a hemostatic seal. In electro-surgical instruments that do not include a harmonic mode (i.e., do not include a harmonic blade), the end effector may be embodied as two clamp arms or jaws. In such embodiments, the electro-surgical instrument may include a separate mechanical knife or blade for cutting the tissue after the creation of the hemostatic seal, which may be incorporated into the elongated shaft attached to the end effector. In bi-polar embodiments, an active electrode may be attached to one of the clamp arms of the end effector and configured to introduce an electrical current into the tissue, which is received by a return electrode attached to the other clamp arm of the end effector (or as the blade itself in embodiments including a harmonic mode). Conversely, in mono-polar embodiments, the return electrode (e.g., a "grounding pad") may be separate from the electro-surgical instrument and located on a different part of the body of the patient. In some embodiments, the electro-surgical instrument may also be configured to apply a sub-therapeutic electrical current to the patient's tissue, which may be used for sensing purposes (e.g., measuring tissue impedance).

Electro-surgery forms hemostatic seals by generating heat in the tissue via the introduced electrical energy, which is embodied as radio frequency ("RF") energy. The particular frequency employed can vary based on the intended use of the electro-surgical instrument within the range of about 100kHz to 1 MHz, although higher frequencies can be employed in some embodiments. Additionally, sub-therapeutic frequencies may be used in some situations for purposes other than hemostatic sealing, such as performing various electrical measurements on the tissue.

It should be appreciated that some energy-based surgical instruments may employ dual or multi-modal technologies for the transection and/or hemostasis of patient tissue. For example, in some cases, an energy-based surgical instrument may include both ultrasonic and electro-surgical capabilities (e.g., by utilizing the ultrasonic blade as an electrode for the electro-surgery mode), which increases the surgical options provided by the surgical instrument to the surgeon.

### SUMMARY

According to one aspect of the disclosure, a system includes an energy-based surgical instrument and a generator coupled to the energy-based surgical instrument. The energy-based surgical instrument includes an end effector having a first electrode and a second electrode, wherein the first electrode includes a first electrode portion and a second electrode portion. The generator is configured to supply a first radio frequency (RF) energy to the first electrode portion for a tissue coagulation process and a second RF energy to the second electrode portion for a tissue transection process, wherein the second RF energy has a higher voltage or current than the first RF energy.

In some embodiments, the generator is configured to supply the second RF energy when a tissue impedance reaches a predetermined impedance range. In some embodiments, the predetermined impedance range comprises 100-160 ohms. In some embodiments, the predetermined impedance range comprises 140-160 ohms.

In some embodiments, the generator is configured to supply the second RF energy when a tissue temperature reaches a predetermined temperature range. In some embodiments, the predetermined temperature range comprises 80-160 °C. In some embodiments, the predetermined temperature range comprises 120-140 °C.

In some embodiments, the generator is configured to supply the second RF energy after supplying the first RF energy.

In some embodiments, the system further includes an insulated coating coupled to the first electrode, wherein the insulated coating increases energy density in a cutting zone of the surgical instrument. In some embodiments, the insulative coating comprises polytetrafluoroethylene (PTFE).

In some embodiments, the end effector further includes a spring bias portion coupled to the first electrode. The spring biased portion is capable of providing elevated pressure at the second electrode portion compared to the first electrode portion. In some embodiments, the spring bias portion further includes a movable portion to maintain a minimum predetermined cutting pressure.

According to another aspect, a method for controlling a surgical instrument includes energizing, by a generator coupled to the surgical instrument, a first electrode portion of a first electrode of an end effector of the surgical instrument with a first radio frequency (RF) energy for a tissue coagulation process; and energizing, by the generator, a second electrode portion of the first electrode with a second RF energy for a tissue transection process, wherein the second RF energy has a higher voltage or a higher current than the first RF energy.

In some embodiments, the method further includes monitoring, by a control element of the surgical instrument, a tissue impedance. Energizing the second electrode portion with the second RF energy comprises energizing the second electrode portion with the second RF energy when the tissue impedance reaches a predetermined impedance range. In some embodiments, the predetermined impedance range comprises 100-160 ohms. In some embodiments, the predetermined impedance range comprises 140-160 ohms.

In some embodiments, the method further includes monitoring, by a control element of the surgical instrument, a tissue temperature. Energizing the second electrode portion with the second RF energy comprises energizing the second electrode portion with the second RF energy when the tissue temperature reaches a predetermined temperature range. In some embodiments, the predetermined temperature range comprises 80-160 °C. In some embodiments, the predetermined temperature range comprises 120-140 °C.

In some embodiments, energizing the second electrode portion comprises energizing the second electrode portion after energizing the first electrode portion.

### BRIEF DESCRIPTION OF THE DRAWINGS

The detailed description particularly refers to the following figures, in which:

The detailed description particularly refers to the following figures, in which:
FIG. 1 is a simplified diagram of an embodiment of a system for performing an energy-based surgical procedure;
FIG. 2 is a perspective view of an embodiment of an energy-based surgical instrument of the system of FIG. 1;
FIG. 3 is a side elevation view of a jaw assembly of an end effector of the surgical instrument of FIG. 2 including an ultrasonic blade and in an open state;
FIG. 4 is a side elevation view of the jaw assembly of the end effector of the surgical instrument of FIG. 2 including an ultrasonic blade and in a closed state;
FIG. 5A is a perspective view of another embodiment of the end effector of the surgical instrument of FIG. 2 including an electrode on a lower jaw clamp of the jaw assembly;
FIG. 5B is a perspective view of another embodiment of the end effector of the surgical instrument of FIG. 2 including two jaw clamps, each having an electrode attached thereto;
FIG. 6 is an exploded view of the surgical instrument of FIG. 2;
FIG. 7 is a block diagram of a control circuit of the surgical instrument of FIG. 2;
FIG. 8 is a perspective view of another embodiment of the end effector of the surgical instrument of FIG. 2 including an electrode attached thereto having several controllable portions; and
FIG. 9 is a simplified flow diagram of at least one method for controlling the surgical instrument of FIG. 8.

### DETAILED DESCRIPTION OF THE DRAWINGS

While the concepts of the present disclosure are susceptible to various modifications and alternative forms, specific illustrative embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the spirit and scope of the invention as defined by the appended claims.

Terms representing anatomical references, such as anterior, posterior, medial, lateral, superior, inferior, distal, proximal, et cetera, may be used throughout the specification in reference to the surgical instruments described herein as well as in reference to the patient's natural anatomy. Such terms have well-understood meanings in both the study of anatomy and the field of surgery. Use of such anatomical reference terms in the written description and claims is intended to be consistent with their well-understood meanings unless noted otherwise.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

The disclosed embodiments may be implemented, in some cases, in hardware, firmware, software, or any combination thereof. The disclosed embodiments may also be implemented as instructions carried by or stored on a transitory or non-transitory machine-readable (e.g., computer-readable) storage medium, which may be read and executed by one or more processors. A machine-readable storage medium may be embodied as any storage device, mechanism, or other physical structure for storing or transmitting information in a form readable by a machine (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

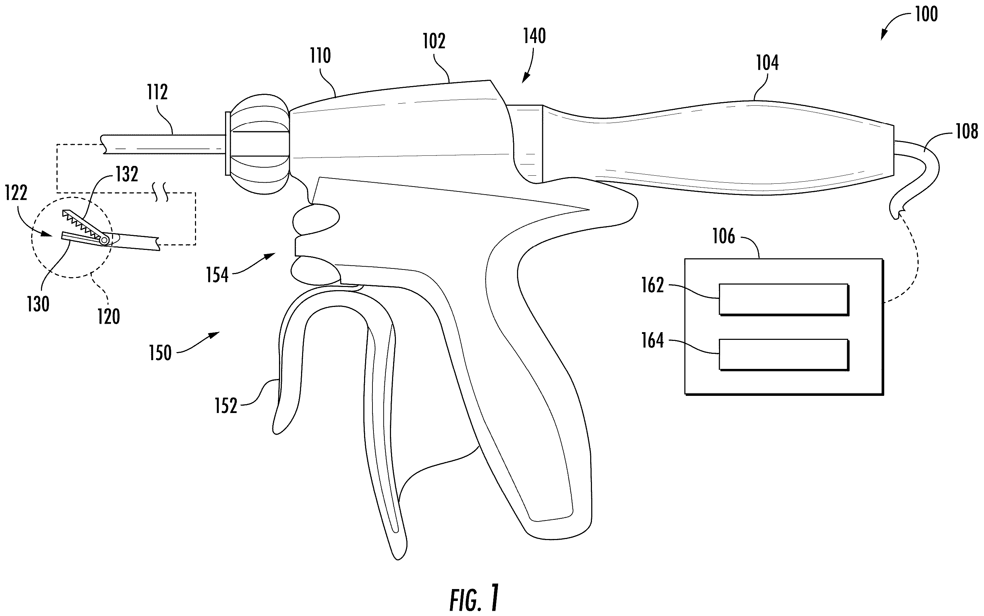

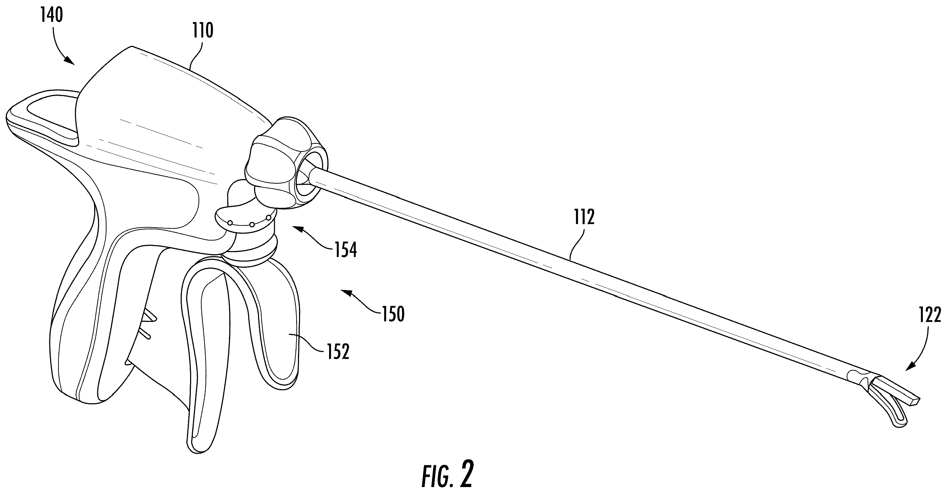

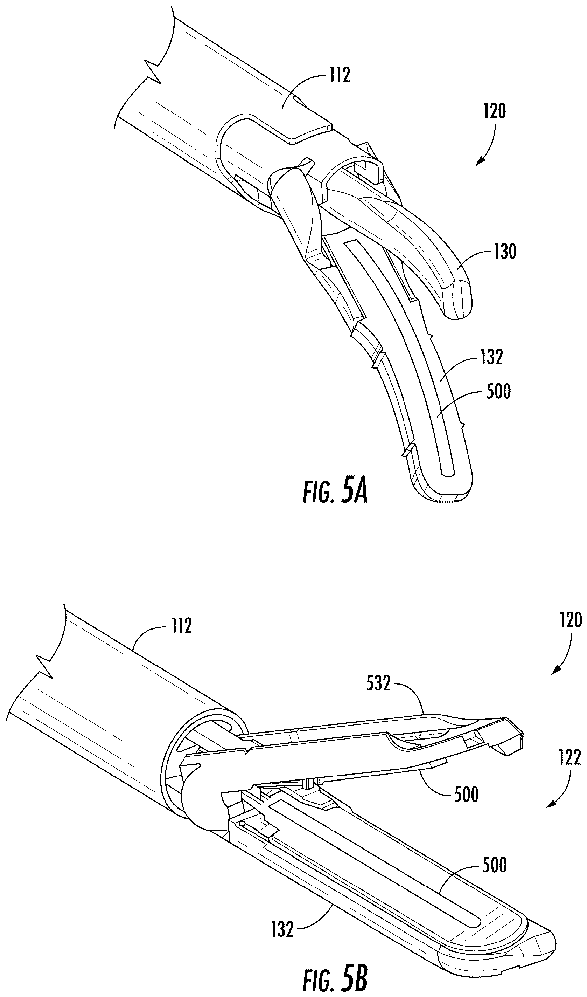

Referring now to FIGS. 1 and 2, in an illustrative embodiment, a system 100 for performing an energy-based surgical procedure includes a surgical instrument 102, a transducer 104, and a generator 106. The surgical instrument 102 is illustratively embodied as an ultrasonic surgical instrument, but may be embodied as an electro-surgical surgical instrument or a multi-modal, ultrasonic/elector-surgical surgical instrument in other embodiments. In use, the surgical instrument 102 is usable to perform various surgical procedures including laparoscopic, endoscopic, or traditional open surgical procedures. In doing so, a surgeon may selectively activate an ultrasonic mode (and/or an electro-surgical/RF mode) of the surgical instrument 102. In the ultrasonic mode, the generator 106 drives the transducer 104 to cause an ultrasonic blade 130 of a jaw assembly 122 of an end effector 120 of the surgical instrument 102 to vibrate at a reference frequency, which facilitates the contemporaneous cutting and hemostatic sealing of patient tissue. Additionally or alternatively, in some embodiments, the surgeon may selectively activate an electro-surgical mode of the surgical instrument 102 to deliver an amount of therapeutic RF energy to the patient tissue to effect hemostatic sealing. In such embodiments, the blade 130 may be embodied as an ultrasonic blade 130 or as a mechanical blade designed to cut tissue using mechanical force (e.g., in those embodiments not employing ultrasonic technologies). Furthermore, in some embodiments, the surgical instrument 102 may be configured with only an electro-surgical/RF mode and, in such embodiments, the jaw assembly 122 of the end effector 120 may not include the ultrasonic blade 130 as discussed in more detail below in regard to FIG. 5B.

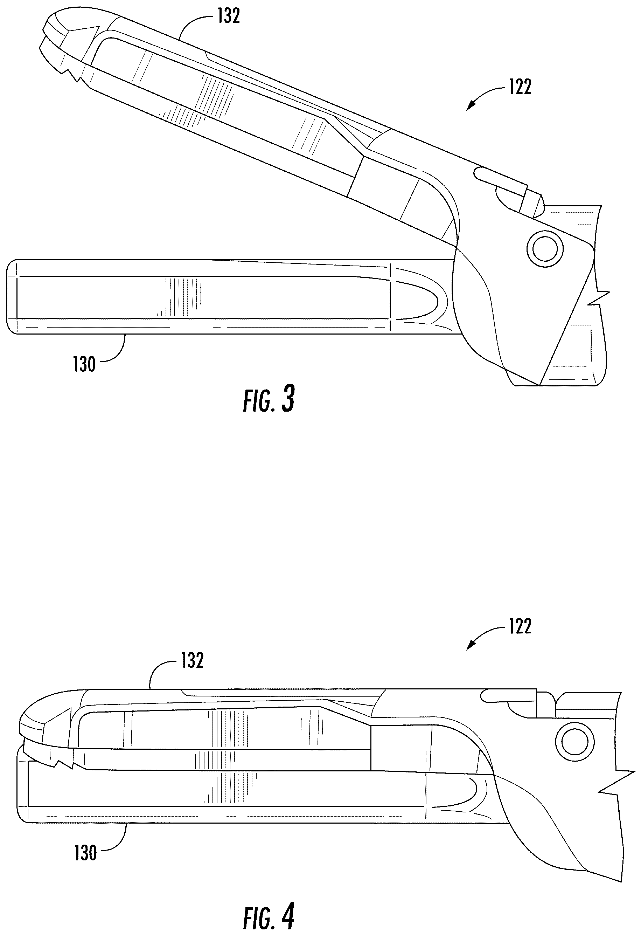

The surgical instrument 102 is illustratively embodied as ultrasonic surgical shears but may be embodied as other types of surgical instruments having an ultrasonic mode and/or electro-surgical mode in other embodiments. In the illustrative embodiment, the surgical instrument 102 includes a handle assembly 110 and an elongated shaft assembly 112, which extends distally away from the handle assembly 110 and may be removably attached to the handle assembly 110 in some embodiments. The elongated shaft assembly 112 includes the end effector 120 located at a distal end opposite the handle assembly 110. The end effector 120 includes the jaw assembly 122, which illustratively includes the ultrasonic blade 130 and a corresponding jaw clamp 132 (but may include two jaw clamps in those embodiments having only an electro-surgical/RF mode). As shown in FIGS. 3 and 4, the jaw assembly 122 is movable between an open state (FIG. 3) in which the jaw clamp 132 is positioned away from the ultrasonic blade 130 and a closed state (FIG. 4) in which the jaw clamp 132 is positioned near or otherwise contacts the ultrasonic blade 130. Actuation of the jaw assembly 122 from the open state to the closed state allows for the grasping, cutting, and coagulation of vessels and/or tissue by the jaw assembly 122. It should be appreciated that the open state may correspond to a degree of openness that is less than a fully opened position of the jaw assembly 122 and the closed state may correspond to a degree of closeness that is less than a fully closed position. That is, the closed state may, for example correspond to a minimal distance between the distal ends of the jaw clamp 132 and the ultrasonic blade 130 and the open state may correspond to a maximum distance between the distal ends of the jaw clamp 132 and the ultrasonic blade 130. However, in other embodiments, the open state may correspond to a fully opened position of the jaw assembly 122 and the closed state may correspond to a fully closed position of the jaw assembly 122.

In those embodiments in which the surgical instrument 102 includes both a ultrasonic mode and an electro-surgical/RF mode, the end effector 120 may include one or more RF electrodes 500 incorporated into the jaw clamp 132 as shown in FIG. 5A. Although the illustrative end effector 120 includes only a single electrode 500 in the embodiment of FIG. 5A, it should be appreciated that the end effector 120 may include additional electrodes 500 in other embodiments (e.g., multiple pads of electrodes 500). The electrode(s) 500 may be embodied as an active electrode configured to the RF energy or as a return electrode configured to "sink" an applied RF energy. In those embodiments utilizing bi-polar RF implementation, the ultrasonic blade 130 may embody the active or return electrode, with the electrode 500 embodying the other active or return electrode. Alternatively, other active or return electrodes may be incorporated on the ultrasonic blade 130 or in another part of the jaw assembly 122 of the end effector 120. In mono-polar implementation, the RF electrode(s) 500 may be embodied as an active electrode, and a return electrode may be attached to a portion of the patient's body.

In those embodiments in which the surgical instrument 102 includes only an electro-surgical/RF mode, the jaw assembly 122 of the end effector 120 includes a jaw clamp 532 in place of the ultrasonic blade 130 as shown in FIG. 5B. In such embodiments, an electrode 500 may be attached to or otherwise incorporated into each jaw clamp 132, 532 and be embodied as an active or a return electrode to facilitate the application of RF energy to tissue captured between the jaw clamps 132, 532. In such embodiments, the surgical instrument 102 may include a knife incorporated into the elongated shaft assembly 112 that is configured to eject outwardly to cut the patient's tissue after sealing of the tissue by the RF energy.

Referring back to FIGS. 1 and 2, in those embodiments including ultrasonic capabilities, the handle assembly 110 includes a receptacle 140 configured to receive the transducer 104 to facilitate connection of the transducer 104 to the handle assembly 110 and the elongated shaft assembly 112. The handle assembly 110 also includes a trigger assembly 150, which includes a primary trigger 152 and a switch assembly 154. The primary trigger 152 is operable by the surgeon to move the jaw assembly 122 of the end effector 120 between the open and closed states. The switch assembly 154 includes one or more buttons, which are selectable by the surgeon to activate (and configure, in some embodiments) the ultrasonic mode and/or the electro-surgical mode of the surgical instrument 102.

The transducer 104 is illustratively connected to the generator 106 by a cable assembly 108. As discussed above, the generator 106 is configured to drive the transducer 104 at a reference or resonant frequency to thereby cause the ultrasonic blade 130 to vibrate. For example, in an illustrative embodiment, the generator 106 may supply an electrical signal to the transducer 104 to cause the ultrasonic blade 130 of the jaw assembly 122 to vibrate longitudinally in the range of, for example, approximately 20 kHz to 250 kHz. In particular embodiments, for example, the ultrasonic blade 130 may vibrate in the range of about 54 kHz to 56 kHz (e.g., at about 55.5 kHz). In other embodiments, the ultrasonic blade 130 may vibrate at other frequencies including, for example, about 31 kHz or about 80 kHz. The excursion of the vibrations at the ultrasonic blade 130 can be controlled by, for example, controlling the amplitude of the electrical signal applied to the transducer 104 by the generator 106. The generator 106 may be activated so that electrical energy may be continuously or intermittently supplied to the transducer 104. The generator 106 also has a power line (not shown) for insertion in an electro-surgical unit or conventional electrical outlet. Additionally or alternatively, the generator 106 may be powered by a direct current (DC) source, such as a battery.

In some embodiments, the generator 106 may be configured to operate in different modes. In such embodiments, the generator 106 may include an ultrasonic generator module 162 for controlling an ultrasonic mode, an electro-surgical/Radio Frequency (RF) generator module 164 for controlling an electro-surgical mode, and/or other generator modules (e.g., a heat generator module) for controlling other operation modes. The various modes of the generator 106 may be operated independently of each other in some embodiments. For example, the generator 106 may activate the ultrasonic mode of the ultrasonic generator module 162 to apply ultrasonic energy to the jaw assembly 122 and subsequently, either therapeutic or sub-therapeutic RF energy may be applied to the jaw assembly 122 by the electro-surgical generator module 164. Alternatively, the activation modes of the generator 106 may be operated simultaneously or contemporaneously with each other.

In the electro-surgical mode, the electro-surgical generator module 164 is configured to generate RF energy at a frequency in the range of about 100 kilohertz (100 kHz) to about 1 megahertz (1 MHz). The generated RF energy is supplied to the patient's tissue via the electrodes 500 of the end effector 120 as described above in regard to FIG. 5. In some embodiments, the electro-surgical generator module 164 may also be configured to selectively provide the RF energy at sub-therapeutic levels to perform various electrical measurements of the patient's tissue. For example, the electro-surgical generator module 164 may be configured to measure an impedance of the patient's tissue using the electrodes 500 and a suitable RF energy level.

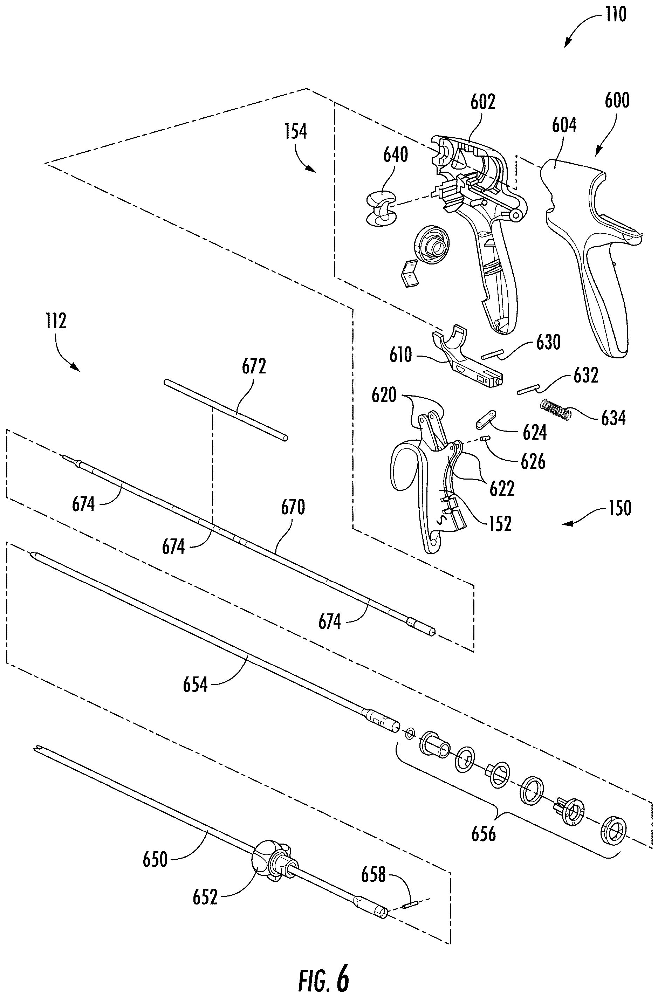

Referring now to FIG. 6, as discussed above, the illustrative surgical instrument 102 includes the handle assembly 110 and the elongated shaft assembly 112, which extends distally away from the handle assembly 110. The handle assembly 110 includes a housing 600, which includes a right half-housing 602 and a left half-housing 604. The half-housings 602, 604 are configured to mate with each other to form the housing 600. To facilitate such mating, each of the half-housings 602, 604 may include various interfaces sized to mechanically align and engage one another to form the housing 600 and enclose the internal working components of the surgical instrument 102.

The primary trigger 152 of the trigger assembly 150 is coupled to a linkage mechanism to translate the rotational motion of the primary trigger 152 to axial motion of a yoke 610, which in turn is configured to move the jaw assembly 122 of the end effector 120 between the open and closed states via the elongated shaft assembly 112. The primary trigger 152 includes a first set of flanges 620 having openings formed therein to receive a first yoke pin 630, which extends through the yoke 610. The primary trigger 152 also includes a second set of flanges 622 configured to receive a first end of a link 624. A trigger pin 626 is received in openings formed in the first end of the link 624 and the second set of flanges 622. The trigger pin 626 forms a trigger pivot point for the primary trigger 152. A second end of the link 624, opposite the first end, is received in a slot formed in a proximal end of the yoke 610 and retained therein by a second yoke pin 632. As the primary trigger 152 is rotated about the pivot point formed from the trigger pin 626, the yoke 610 translates horizontally. A spring 634 is used to bias the yoke forward such that the jaw assembly 122 of the end effector 120 is biased to the open state (or a fully opened state).

As discussed above, the trigger assembly 150 also includes a switch assembly 154. The switch assembly 154 illustratively includes a toggle switch 640, which is selectable to activate one or more switches 642. Activation of the switches 642 electrically energizes an electrical element 644, which electrically energizes the ultrasonic transducer 104 to engage the ultrasonic mode of the surgical instrument 102.

The elongated shaft assembly 112 includes an outer tubular sheath 650 and a rotation knob 652 coupled to the outer cylindrical sheath 650. The rotation knob 652 is operable to rotate the outer cylindrical sheath 650 about an axis defined by the outer cylindrical sheath 650. A reciprocating tubular actuator 654 is located within the outer tubular sheath 650 and mechanically engaged with the end effector 120 on a distal end. The reciprocating tubular actuator 654 is also mechanically engaged, on a proximal end, with the yoke 610 within the handle assembly 110 via coupling elements 656. In embodiments including an ultrasonic mode, an ultrasonic waveguide 670 is located within the reciprocating tubular actuator 654. A distal end of the ultrasonic waveguide 670 is acoustically coupled (e.g., directly or indirectly mechanically coupled) to the ultrasonic blade 130, and a proximal end is acoustically coupled to the transducer 104. The ultrasonic waveguide 670 may be isolated from other components of the elongated shaft assembly 112 by a protective sheath 672 and a number of isolation elements 674. The outer tubular sheath 650, the reciprocating tubular actuator 654, and the ultrasonic waveguide 670 are mechanically engaged together via a pin 658.

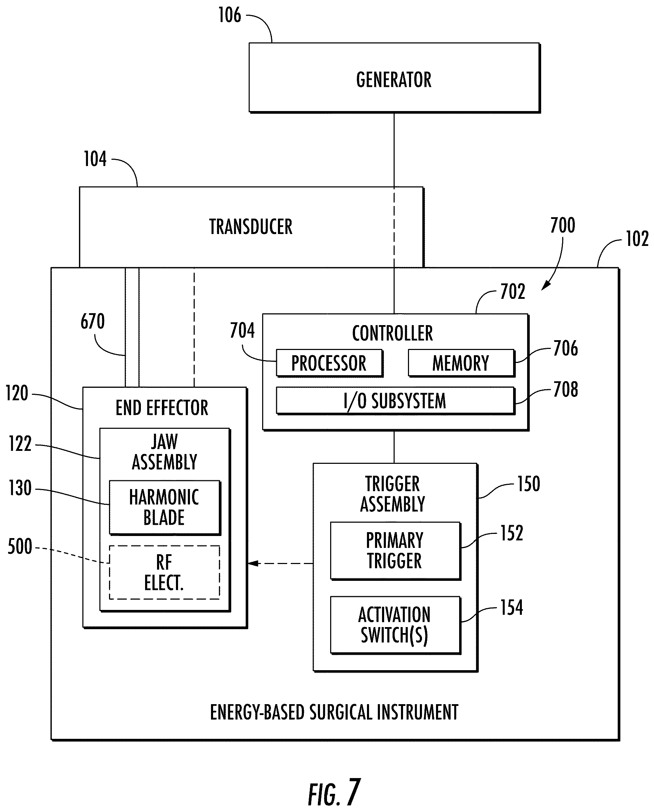

Referring now to FIG. 7, in the illustrative embodiment, the surgical instrument 102 includes a control circuit 700. The control circuit 700 includes a controller 702 and the trigger assembly 150, which cooperate to provide ultrasonic energy to the harmonic blade 130 of the jaw assembly 122 of the end effector 120 and/or RF energy to the RF electrodes 500 of the jaw assembly 122, depending on the operation modes of the surgical instrument 102 as discussed above. In other embodiments, however, the control circuit 700 may include additional or other electronic devices and/or circuit.

The controller 702 may be embodied as any type of controller, functional block, digital logic, or other component, device, circuitry, or collection thereof capable of performing the functions described herein. In illustrative embodiment, the controller 702 includes a processor 704, a memory 706, and an input/output (I/O) subsystem 708. The processor 704 may be embodied as any type of processor capable of performing the functions described herein. For example, the processor 704 may be embodied as a single or multi-core processor(s), digital signal processor, microcontroller, or other processor or processing/controlling circuit. Similarly, the memory 706 may be embodied as any type of volatile and/or non-volatile memory or data storage capable of performing the functions described herein. In operation, the memory 706 may store various data and software used during operation of the control circuit 700 such as executable firmware or software, programs, libraries, and drivers, which may be executed or otherwise used by the processor 704.

The processor 704 and memory 706 are communicatively coupled to other components of the control circuit 700 via the I/O subsystem 708, which may be embodied as circuitry and/or components to facilitate input/output operations between the controller 702 (e.g., the processor 704 and the memory 706) and the other components of the control circuit 700. For example, the I/O subsystem 708 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations. In some embodiments, the I/O subsystem 708 may form a portion of a system-on-a-chip (SoC) and be incorporated, along with the processor 704 and the memory 706, and other components of the surgical instrument 102, on a single integrated circuit chip. Additionally, in some embodiments, the memory 706, or portions of the memory 706, may be incorporated into the processor 704.

During operation, as discussed above, the controller 702 is configured to control activation of an ultrasonic mode and/or an electro-surgical/RF mode of the surgical instrument 102. To do so, the controller 702 may monitor for activation of the primary trigger 152 and/or one or more activation switches 154 of the trigger assembly 150. In response to activation of the appropriate trigger 152 or switch 154, the controller 702 controls the transducer 104 to generate the ultrasonic energy, which is propagated to the harmonic blade 130 via the ultrasonic waveguide 670. Additionally or alternatively, in response to activation of a corresponding switch 154 of the trigger assembly 150, the controller 702 may be configured to supply an amount of RF energy, via the electro-surgical generator module 164 to the RF electrodes 500 via interconnections 710. It should be appreciated that, although the transducer 104 and the generator 106 are shown as separate components from the energy-based surgical instrument 102 in FIGS. 1 and 7, the transducer 104 and/or the generator 106 may be incorporated into the surgical instrument 102 in other embodiments.

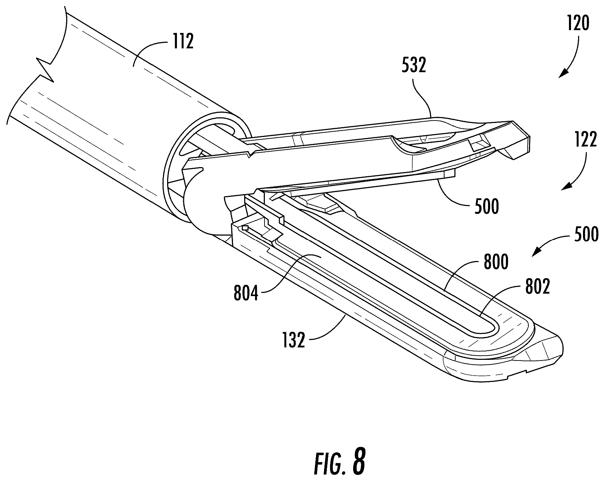

Referring now to FIG. 8, in another illustrative embodiment, an end effector 120 for a surgical instrument includes a jaw assembly 122 including an electrode 500 attached to each of a jaw clamp 132, 532, similar to the end effector 120 shown in FIG. 5B and described above. In the illustrative end effector 120 shown in FIG. 8, the electrode 500 attached to the jaw claim 132 includes multiple portions 800, 802, which are surrounded by and/or embedded in a nonconductive tissue pad 804.

Each of the electrode portions 800, 802 may be supplied with a separate amount or waveform of energy while performing a cutting operation or in a cutting zone of the electrode. This separate bipolar energy could be of a higher voltage or current than the energy supplied to a primary coagulation portion of the electrode 500 set. Of course, a different number and/or arrangement of electrode portions 800, 802 may be used in other embodiments.

Additionally or alternatively, each separate electrode 500 may have insulated coatings on portions of the electrode 500 that would further concentrate energy density within the cutting zone to a further constrained version. For example, a portion of the cutting electrode 500 may be coated with a thick layer of polytetrafluoroethylene (PTFE), creating a finer focal area for the energy to be transferred to the tissue, while further creating an inhibited sticking portion of the retainer of the electrode 500, which may minimize the accumulation of char or debris.

Additionally or alternatively, the cutting electrode may have geometry integral to the conductive portion that creates a small thin energy focal zone relative to the rest of the cutting electrode. The portion of the geometry outside of that thin focal area may have an insulative coating to further minimize the energy density zone and also prevent tissue accumulation.

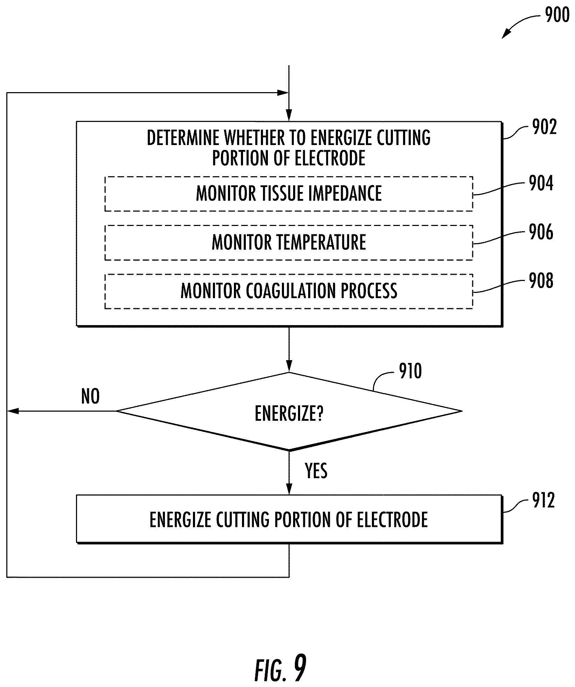

Referring now to FIG. 9, a method 900 for controlling an energy-based surgical instrument 102 is shown. The method 900 may be executed by the controller 702, the generator 106, and/or one or more other microcontrollers or other control elements of the system 100. The method 900 begins in block 902, in which the control element determines whether to energize a cutting portion of an electrode 500 of the surgical instrument 102. The cutting portion may be embodied, for example, as the central portion 800 of the electrode 500 shown in FIG. 8, above, or another electrode portion positioned in a cutting zone. The cutting electrode portion may be energized, for example, as part of the primary coagulation cycle (e.g., energizing after the beginning of the coagulation cycle but before the end of the cycle), or the cutting electrode portion could be energized after the coagulation cycle is complete.

In some embodiments, in block 904 the control element may monitor tissue impedance to determine whether to energize the cutting electrode portion. For example, if the cutting operation is done as part of the primary coagulation cycle, the energizing of the separate electrode portion may be activated based on a tissue impedance, for example activated when measured tissue impedance is between 100-160 Ω, or as another example when measured tissue impedance is between 140-160 Ω. In some embodiments, in block 906 the control element may monitor temperature (e.g., tissue temperature or instrument temperature) to determine whether to energize the cutting electrode portion. For example, if the cutting operation is done as part of the primary coagulation cycle, the energizing of the separate electrode portion may be activated based on temperature (e.g., tissue temperature or instrument temperature), for example activated when measured temperature is between 80-160 °C, or as another example when the measured temperature is between 120-140 °C. In some embodiments, in block 908 the control element may otherwise monitor coagulation process. For example, the control element may energize the cutting portion when the coagulation cycle is completed, which may be determined based on an elapsed time, change in operating mode, or other indication of the coagulation process.

In block 910, the control element checks whether to energize the cutting electrode portion. If not, the method loops back to block 902 to continue determining whether to energize the cutting electrode portion. If so, the method 900 advances to block 912. In block 912, the control element energizes the cutting portion of the electrode. As described above, the cutting electrode portion may be energized with a separate bipolar energy, which may be of a higher voltage or current than the energy supplied to a primary coagulation portion of the electrode 500 set. This higher cutting energy may be further focused or concentrated by one or more insulated coatings or other non-conductive features. After energizing the cutting electrode portion, the method 900 loops back to block 902 to continue determining whether to energize the cutting electrode portion.

Additionally, or alternatively, in some embodiments the end effector 120 may include a pressure control system, such as a spring bias support, which may be positioned underneath part or all of the electrode portions 800, 802 and/or the tissue pad 804 shown in FIG. 8. Accordingly, in those embodiments, the support surface for the concentrated cutting energy density zone has a spring biased portion which is capable of providing elevated pressure over the rest of the coagulation electrode zone. Additionally, the support surface may also have a deflectable, deformable or movable portion, where the higher pressure zone is insured but it does not prevent the rest of the electrode gap to be different due to the differences of tissue thickness in the cutting portion. As the tissue softens, thins, or is disrupted, the spring bias returns the support to a closer approximation to the cutting electrode in order to maintain a minimum predefined cutting pressure.

While the disclosure has been illustrated and described in detail in the drawings and foregoing description, such an illustration and description is to be considered as illustrative and not restrictive in character, it being understood that only illustrative embodiments have been shown and described and that all changes and modifications that come within the spirit of the disclosure are desired to be protected.

There are a plurality of advantages of the present disclosure arising from the various features of the methods, apparatuses, and systems described herein. It will be noted that alternative embodiments of the methods, apparatuses, and systems of the present disclosure may not include all of the features described yet still benefit from at least some of the advantages of such features. Those of ordinary skill in the art may readily devise their own implementations of the methods, apparatuses, and systems that incorporate one or more of the features of the present invention and fall within the spirit and scope of the present disclosure as defined by the appended claims.

## Claims

1. A system comprising:
an energy-based surgical instrument comprising an end effector having a first electrode and a second electrode, wherein the first electrode comprises a first electrode portion and a second electrode portion; and
a generator coupled to the energy-based surgical instrument, wherein the generator is configured to supply a first radio frequency (RF) energy to the first electrode portion for a tissue coagulation process and a second RF energy to the second electrode portion for a tissue transection process, wherein the second RF energy has a higher voltage or a higher current than the first RF energy.

2. The system of claim 1, wherein the generator is configured to supply the second RF energy when a tissue impedance reaches a predetermined impedance range.

3. The system of claim 1 or claim 2, wherein the generator is configured to supply the second RF energy when a tissue temperature reaches a predetermined temperature range.

4. The system of any preceding claim, wherein the generator is configured to supply the second RF energy after supplying the first RF energy.

5. The system of any preceding claim, further comprising an insulated coating coupled to the first electrode, wherein the insulated coating increases energy density in a cutting zone of the surgical instrument, preferably wherein the insulative coating comprises polytetrafluoroethylene (PTFE).

6. The system of any preceding claim, wherein the end effector further comprises a spring bias portion coupled to the first electrode, wherein the spring biased portion is capable of providing elevated pressure at the second electrode portion compared to the first electrode portion, preferably wherein the spring bias portion further comprises a movable portion to maintain a minimum predetermined cutting pressure.

7. A method for controlling a surgical instrument, the method comprising:
energizing, by a generator coupled to the surgical instrument, a first electrode portion of a first electrode of an end effector of the surgical instrument with a first radio frequency (RF) energy for a tissue coagulation process; and
energizing, by the generator, a second electrode portion of the first electrode with a second RF energy for a tissue transection process, wherein the second RF energy has a higher voltage or a higher current than the first RF energy.

8. The method of claim 7, further comprising:
monitoring, by a control element of the surgical instrument, a tissue impedance;
wherein energizing the second electrode portion with the second RF energy comprises energizing the second electrode portion with the second RF energy when the tissue impedance reaches a predetermined impedance range.

9. The system of claim 2 or the method of claim 8, wherein the predetermined impedance range comprises 100-160 ohms, preferably 140-160 ohms.

10. The method of claim 7, further comprising:
monitoring, by a control element of the surgical instrument, a tissue temperature;
wherein energizing the second electrode portion with the second RF energy comprises energizing the second electrode portion with the second RF energy when the tissue temperature reaches a predetermined temperature range.

11. The system of claim 3 or the method of claim 10, wherein the predetermined temperature range comprises 80-160 °C, preferably 120-140 °C.

12. The method of any one of claims 7 to 11, wherein energizing the second electrode portion comprises energizing the second electrode portion after energizing the first electrode portion.
